# EUROPEAN PATENT SPECIFICATION

(11) **EP 4 114 571 B1**
(45) Date of publication and mention of the grant of the patent: **01.01.2025**
(21) Application number: 22710631.7
(22) Date of filing: 10.03.2022
(51) Int. Cl.: B01L 3/00, G01N 33/53, G01N 33/574

(54) **QUANTIFICATION OF CELL MIGRATION AND METASTATIC POTENTIAL OF TUMOR CELLS**
QUANTIFIZIERUNG DER ZELLMIGRATION UND DES METASTATISCHEN POTENTIALS VON TUMORZELLEN
QUANTIFICATION DE LA MIGRATION CELLULAIRE ET DU POTENTIEL MÉTASTATIQUE DES CELLULES TUMORALES

(30) Priority: 10.03.2021 EP 21161887
(43) Date of publication of application: 11.01.2023
(73) Proprietor: Cline Scientific AB, 413 53 Mölndal (SE)
(72) Inventor: SUNDH, Patrik, 423 41 Torslanda (SE); EVENBRATT, Hanne, 443 95 Stenkullen (SE); HOLMQUIST, Niklas, 434 91 Kungsbacka (SE); ANDÄNG, Michael, 146 54 Tullinge (SE); VINCENT, Theresa, 114 42 Stockholm (SE); SZESZULA, Petra, 134 62 Ingarö (SE); MOBINI, Reza, 413 08 Göteborg (SE)
(74) Representative: Ström & Gulliksson AB
(86) International application number: PCT/EP2022/056177
(87) International publication number: WO 2022/189565

(56) References cited:
- EP-A2- 2 057 277
- EP-B1- 2 057 277
- US-A1- 2002 028 451
- US-A1- 2018 172 694
- US-A1- 2020 164 375

## Description

### FIELD OF THE INVENTION

The present disclosure relates to the field of live cell culture, the quantification of cell migration, and the metastatic potential of tumor cells.

### BACKGROUND

Cancer is the second largest worldwide cause of death and poses the highest clinical, social, and economic burden among all human diseases. The majority of cancer deaths are caused by metastases, where the tumor cells have detached from the primary tumor and spread to distant organs.

Existing solutions to predict the outcome of breast tumors and the likelihood of metastasis have emerged in recent years. These include classical pathology (e.g., histopathology and MRI/PET scan), and biomarkers (e.g., the presence of receptors for estrogen (ER), progesterone (PR), and human epidermal growth factor receptor 2 (HER2)). However, classical pathology only detects metastases in advanced stages and while biomarkers are used to classify breast cancer and provide information on whether hormonal treatment would be advantageous and prediction of recurrence, they do not directly determine metastatic potential. Significant efforts have been made to develop high-throughput molecular technologies to identify molecular markers such as gene signatures to guide decision-making processes (e.g., PAM50, MammaPrint). New biomarkers aim at discovering metastasis but are limited by detecting a single or a small set of genes or proteins, which provides only indirect indication of potential metastasis in specific subclasses of breast cancer. Moreover, interpretation and clinical translation of results from multiple genomic and proteomic analysis variables remain a significant challenge. Genetic testing can be expensive, time-consuming, and limited due to the biasing presence of normal cells and intratumoral heterogeneity. These methods have the drawback of only providing a snapshot of different factors and do not quantify the real-time migration and behavior of living cells.

Ideally, reliable diagnostics could help to distinguish invasive tumor cells and, subsequently, to define appropriate treatment strategies at an early stage. However, a method that can definitively and efficiently determine whether a primary tumor will metastasize does not exist today and the ability to tailor the patient's therapy is therefore limited. Further, the ability to predict and understand the mechanisms of metastasis would be of great value in the search for metastasis-limiting pharmaceutical therapies.

Chemotaxis studies have been crucial for the study of cell migration and tumor metastasis. One way to investigate and predict intratumoral cells' metastatic behavior is with the use of biomolecular gradients. Biomolecular gradients are established by a difference in the concentration of molecules in a biological system and are important for communication, adhesion, and morphology of cells. Different methods to provide chemical gradients have been established over the years. However, they are limited in their ability to produce precise and consistent enough controlled gradients for such studies.

Traditional methods of producing biomolecule gradients (gradient chambers, micropipette-generated, biological hydrogel) have been useful instrumentals in scientific research, however, they are limited in their ability to produce precise and quantitative results necessary for a diagnostic product. Biomolecule gradients constructed in free liquid solutions can behave inconsistently and diffuse over time and are highly susceptible to small vibrations, thermal imbalances, or evaporation. For biological hydrogel-based gradients, the use of cell-based biomolecule sources such as explants or transfected cell lines to form gradients leads to high variability in resultant gradients (Keenan TM, Folch A. Biomolecular gradients in cell culture systems. Lab Chip. 2008 Jan;8(1):34-57. doi: 10.1039/b711887b. Epub 2007 Dec 6. PMID: 18094760; PMCID: PMC3848882). These limitations mean that cells are not exposed to a constant, precise user-defined gradient and results are often not quantitative and not of adequate precision, both of which necessary for a diagnostic product.

Moreover, existing cell migration assays do not replicate the real-life tumor conditions and are therefore not an accurate representation of tumor cells in vivo. Further, they are not able to combine multiple factors and biomolecule gradients, a key competent in order to replicate *in vivo* cellular conditions.

In recent years microfluidic technology has been developed for chemotaxis and cell migration studies and a proposed way to overcome the above noted limitations. An example of this is US 2018 0 280 976, which relates to gradient-on-a-chip, which discloses a cell culture device and its applications of creating gradient of chemicals or gradient of cells to mimic the in vivo physiological conditions of homeostasis.

Some work has been done to apply the technology to determine metastatic potential of cells derived from cancer patients. Examples include US 2016 0 158 751 and US 2020 0 249 232 which discloses the use of an integrated microfluidic chip for analysis of cell motility and prediction and prognosis of patient survival, with or without a chemical gradient.

US 2018/0172694 A1 discloses sensor arrays for detecting biomolecules and methods of use. The plurality of sensor elements in the sensor arrays comprises, consists essentially of, or consists of a plurality of particles. Each particle is differentiated from each other by at least one physicochemical property such that each sensor element has a unique biomolecule corona signature when placed in contact with the same sample. The size of the particles in different sensor elements may thus differ to affect the binding selectivity. There is, however no variation in average inter-particle distance. A variation in the inter-particle distance may provide variation in the binding selectivity for a given sensor element.

US 2002/0028451 A1 discloses a detection apparatus for use in the detection of the presence of a selected pathogen in a sample. Such apparatus include: a substrate with a detection region on a surface thereof, the detection region having microstructures including grooves formed therein that will align liquid crystal material in contact therewith, the width and depth of the grooves being in the range of 10 µm or less; a blocking layer on the surface of the detection region of the substrate that does not disrupt the alignment of liquid crystal material in contact therewith, the blocking layer blocking nonspecific adsorption of pathogens to the surface; and a binding agent on the surface of the detection region of the substrate, the binding agent specifically binding the selected pathogen. The detection region may be coated with a continuous metallic layer, e.g., a silver or a gold film, applied by vacuum deposition.

EP 2 057 277 describes methods and compositions based on liquid crystal assays and other biophotonically based assays for detecting and quantifying the number of cells present on a test surface or within a test substrate and the proliferation, death, or movement of cells under control conditions and in response to chemotactic and other cytoactive (including compounds that are chemokinetic but not chemotactic and agents that inhibit cell migration) agents. In order to provide wells on the test surface that are isolated from each and not being in fluid communication with each other, particles may be deposited on the test surface in patterns to provide raised walls or partitions. In this manner, an analyte (e.g., a potential chemotactic agent) placed in a particular well remains substantially confined to that well. Further, patterning may allow the creation of microchannels through the device whereby an agent can exit such that e.g., diffusion gradients for testing whether a test compound is a chemotactic agent, i.e., whether the direction of movement of motile cells or organisms are affected by a diffusion gradient of a diffusible test compound.

However, these newer methods also have drawbacks. Disadvantages of microfluidics-based methods include their cost, complexity, and that the forces and pressures present could be disturbing or damaging to cells. These are still subject to inconsistency in gradient and conditions for the cells due to the fluidic nature of the apparatuses.

Thus, there exists a need for a more effective device and method for measuring tumor cell migration and determine the metastatic potential of tumors cells.

### SUMMARY

An object of the disclosure is to provide a method and device that can be used to measure migration capacity, i.e., the movement of a cell, and metastatic potential of tumor cells in vitro.

According to a first aspect of the disclosure, these and other objects are achieved, in full or at least in part, by a gradient-on-a-chip device for quantification of cell migration and metastatic potential of tumor cells. The device comprises:
- a chip having a chip surface;
- a nano gradient layer of nanoparticles provided on the chip surface, the nano gradient layer having a gradient direction along an axis of an X-Y plane of the chip surface; and
- a biomolecule conjugated to the nanoparticles by means of a linker linking together said biomolecule to said nanoparticles (50);

The chip surface further has at least one guiding structure arranged to guide the tumor cells in the gradient direction. The guiding structure extends in the gradient direction and delineates a migration corridor comprising the nano gradient layer.

Nano gradients constitute a useful tool to induce cell migration. It was envisaged that surface-bound nano-gradient may overcome many of the issues in the field as they provide a precise and controllable continuous gradient. A preferred type of a precise and controllable continuous gradient is disclosed in WO 2012/025576.

The nano gradient layer, according to the invention, is described as a dispersion of nanoparticles onto a chip surface in a gradient pattern, where the density of particles increases along one axis of the chip surface. The nano gradient layer may for example consist of nano-patterns of gold nanoparticles, onto which biomolecules of interest are attached. It is created to mimic tissue function at the molecular level, e.g., intra-tumoral spaces. The gradient layer is utilized to influence and direct cell migration along the gradient direction of the nano gradient layer. It may also be utilized to screen for sensitive reactions and/or interactions between cells and biomolecules, thus determining the optimal composition of biomolecules responsible for a certain cellular response. The region on the gradient responsible for a reaction may then be recreated in a nano layer. A method for producing the nano gradient layer is described in WO 2012/025576.

However, the measurement of cell migration in a nano gradient layer can be problematic due to interactions between cells. Such cell-cell interactions occur when adjacent cells get in contact, and often causes the cells to change its migration path, possibly in a direction opposite the gradient direction of the nano gradient. The cell-cell interactions thus disturb the migration behavior of the cells. Furthermore, cell-cell interactions may complicate screening and analyzation of the interactions between cells and biomolecules.

The more cells present in a specified area, the greater risk that disturbing cell-cell interactions will occur when the cells starts to migrate. Measurements has shown that severe cell-cell interactions may disturb cell migration to such an extent that it is difficult, if not even possible, to retain accurate analyzation results of cells migration behavior. Cell-cell interaction may also cause disturbance when an optimal composition between cells and biomolecules is to be determined.

The inventors have solved this problem by a guiding structure arranged in the gradient direction. The guiding structure extending in the gradient direction is meant to be understood as the guiding structure extending substantially parallel with the gradient direction.

The function of the guiding structure is to delineate a migration corridor for the cells. Hereby, the migration corridor is delimited by the guiding structure. A migration corridor is defined as the space between the guiding structure and the outer edge of the chip surface. A device having only one single guiding structure thus contains two migration corridors, the two of them located on each side of the guiding structure. Further, also the space located between two adjacent and parallel guiding structures defines a migration corridor. According to an example, the migration corridor is delineated by two adjacent and parallel guiding structures.

The migration corridor is adapted to guide the cells in the gradient direction along the nano gradient layer and to prevent cells from interacting with each other. Hereby, the area for each cell to migrate in is delimited. The device thus has the advantage that it eliminates disturbance from cell-cell interactions.

Moreover, the migratory behavior of tumor cells within a migration corridor may be more correctly and reliantly measured and recorded than without migration corridors, which enhances the capability of predicting cells' ability to spread and metastasize. A further advantage is that the time for measurement may be shortened when cell-cell interactions are avoided.

According to one embodiment, the nanogradient layer is a continuous nanogradient layer. This means the concentration of nanoparticles increases continously in the gradient direction.

According to at least one example embodiment, the guiding structure is a ridge extending out of the chip surface or a recession pointing into the chip surface. Such a ridge may be 1 to 100 µm high, such as 10 to 30 µm high. Further non-limiting examples of guiding structures are cuts, scores, notches, or incisions in the chip surface. These examples illustrate guiding structures that are contained in the chip surface. The depth of such guiding structures may be 1 to 100 µm, such as 10 to 30 µm. Alternatively, the chip surface may be modified with excess material to create a guiding structure in the form of a ridge or a wall. The guiding structure may contain the same material as the chip and/or a different material. Further, the width of the guiding structure may be at least 1 µm, such as 1 to 900 µm, or 5 to 500 µm. The width may be measured in the plane from which the guiding structure extends out of, or into, the chip surface.

According to at least one example embodiment, the guiding structure extends continuously along the chip surface. This is to be interpreted as the guiding structure extending continuously along the migration corridor. Hereby, the delineation of the migration corridor is continuous along the gradient direction of the chip surface.

In an embodiment, the guiding structure extends continuously along a portion of the chip surface. Alternatively, the guiding structure may extend discontinuously along the corridor. There may for example be breaks in the guiding structure along the gradient direction.

According to at least one example embodiment, the device further comprises a chip surface area void of nano gradient layer, and wherein the guiding structure is the boundary line between the migration corridor and the chip surface area void of nano gradient layer.

This is to be interpreted as the migration corridor being delimited by a surface area void of nano gradient layer. An area void of nano gradient layer also means an area void of biomolecules. Such an area is not pleasant for the cells to migrate into, or uphold themselves in, as they prefer to interact with the biomolecules and thus follow the gradient direction.

According to at least one example embodiment, the migration corridor has a substantially constant width along its extension direction.

The migration corridor has thus equal width in both end points. By having a constant width along the extension direction, the cells are induced to travel linearly in the gradient direction.

According to the present invention, the migration corridor has a width in the range of 20 to 500 µm, and preferably 50 to 200 µm.

According to an example, the migration corridor has a width in the range from 30 to 120 µm, more preferably 40 to 80 µm, and most preferably 50 to 60 µm.

Hereby, the width of the corridor matches a cell size of about 50 µm. By having a corridor width matching the size of the cell, linear cell migration may be induced. A corridor having the width of 50 to 60 µm, e.g., about 50 µm, is preferably used for the measurement of a single cell or a low number of cells, e.g., cells that have been loaded at different loading areas along the gradient direction. A wide corridor has the advantage that a plurality of cells may be present in the same corridor. Whereas one typically would like to minimize cell interactions, there may still be some types of experiments in which it would be of interest to have a plurality of cells present in the same corridor.

The migration corridor is 1 to 20 mm long. The corridor may be about 5 mm long, such as 1 to 10 mm long, or 2 to 8 mm long. According to the present invention, the migration corridor has length:width ratio of 200:1 to 20:1

According to at least one example embodiment, the gradient-on-a-chip device comprises two or more migration corridors. Two or more migration corridors allow simultaneous measurement of the migration behavior of a plurality of cells. This is beneficial since it saves time for analyses. The more migration corridors that are present, the more cells may be measured simultaneously.

According to at least one example, the gradient-on-a-chip device comprises at least 50 migration corridors, at least 100 migration corridors, or at least 150 migration corridors. The number of migration corridors may be correlated to the size of the chip. A large chip has the benefit that more corridors may be present than on a small chip.

According to an example, the device comprises two or more guiding structures.

According to an example, the device comprises at least 25 guiding structures, preferably at least 50 guiding structures, and most preferably at least 100 guiding structures.

According to at least one example, the two or more migration corridors are of equal width.

This may be achieved by providing a single guiding structure provided in the center of the chip surface, the guiding structure delineating two separate migration corridors provided on each side of the guiding structure.

An advantage of having two or more migration corridors of equal width is the results of different cells can be compared. A further advantage may be that the manufacturing could be easier and cheaper.

The device may comprises two or more migration corridors of different widths.

According to an embodiment, the biomolecule is an epidermal growth factor (EGF). Other non-limiting examples of biomolecules are Semaphorin-3E (Sema3E), Semaphorin-4D (Sema4D), Semaphorin-5a (Sema5a), C-C motif chemokine 27 (CCL27), C-C motif chemokine 38 (CCL38), C-C motif chemokine 48 (CCL48), C-C motif chemokine 58 (CCL58), C-C motif chemokine 12 (CCL12), C-C motif chemokine 199 (CCL199), C-C motif chemokine 21 (CCL21), C-C motif chemokine 22 (CCL22), C-C motif chemokine 25 (CCL25), C-X-C motif chemokine 5 (CXCL5), C-X-C motif chemokine 8 (CXCL8) (IL-8), C-X-C motif chemokine 9 (CXCL9), C-X-C motif chemokine 10 (CXCL10), C-X-C motif chemokine 12 (CXCL12), C-X-C motif chemokine 13 (CXCL13), C-X-C motif chemokine 14 (CXCL14), Interleukin-11 (IL-11), Fibroblast growth factor (FGF), Platelet-derived growth factor (PDGF), Placenta growth factor (PIGF), Hepatocyte growth factor (HGF), HB-EGF (Heparin-binding, EGF-like), Slit homolog 2 protein (Slit2), Vascular endothelial growth factor a (Vegf-a), Vascular endothelial growth factor b (Vegf-b), Vascular endothelial growth factor c (Vegf-c), Ephrin type-A receptor 2 (EphA2) (Eph - receptor), and Ephrin type-B receptor 4 (EphB4). In regard to mimicking the intratumoral environment, these biomolecules may be combined with ECM fibers such as fibronectin, collagen, elastin, or laminin.

In one embodiment, the chip surface between the nanoparticles, or at least a part of the chip surface between the nanoparticles, is coated by a coating agent, such as ECM fibers. An example of coating agent is fibronectin. Other examples of coating agents are laminin, collagen and elastin.

According to at least one example embodiment, the linker comprises the linker complex biotin/streptavidin. In such an embodiment, streptavidin may be attached to the particle, whereas biotin is attached to the biomolecule.

As already described, the device comprises a chip with a chip surface. A gradient of nanoparticles is provided on the chip surface. The particles may have a diameter in the range 1 to 100 nanometers (nm), such as in the range 3 to 30 nanometers (nm). Exemplary particles have a diameter of about 10 nanometers (nm). Other exemplary particles have a diameter of about 5 nanometers (nm) or about 20 nanometers (nm). Further, the average center-to-center distance between the nanoparticles may be 10 to 60 nm, such as 15 to 50 nm, in one end of the gradient and 100 to 150 nm in the other end of the gradient. The center-to-center distance between the nanoparticles is longer than the particle diameter, such as at least 10% longer than the particle diameter. According to an embodiment, at least 95% of the particles have a diameter in the stated range. According to an embodiment, at least 95%, such as at least 97.5%, of the particles have a diameter in the range 3 to 25 nm.

In one embodiment, the nanoparticles are gold particles. The gold particles may be coated with thiolated streptavidin. Further, the biomolecules may be biotinylated and attached to the nanoparticles through biotin/streptavidin interaction. The gold particles may have a diameter of 3 to 25 nm, such 5 to 20 nm. Exemplary gold particles may have a diameter of about 5±1, 10±1.5, or 20±4 nm.

However, any such known principle for facilitating binding and interaction between nanoparticles and a biomolecule may be used since the biotin/streptavidin interaction is only an interaction according to one embodiment. For instance, such interaction may be formed using an ion-exchange/interaction, hydrophobic interactions and dative binding, covalent binding of thiols, carbo di-imide chemistry, bi-functional linkers (such as EDC/NHS chemistry) or click chemistry.

According to at least one example, device further comprises two or more migration corridors provided on top of each other. This may be achieved by two chips, each with a nano gradient layer, provided on top of each other.

According to a second aspect of the disclosure, a method for quantification of cell migration and metastatic potential of tumor cells is provided, as claimed in claim 11.

Measuring and/or recording means that the position of the tumor cell on the chip surface is determined at various points in time. By this, the migration behavior of the cells may be documented. Non-limiting examples of features that may be determined are migration velocity, migration distance, migration motility and migration direction. Based on such features, cell migration may be quantified and in turn, the metastatic potential of tumor cells.

The method according to the inventive concept has the advantage that it may generate results within hours, therefore saving time and costs associated with diagnosis. The added information about the nature of the tumor behavior and risk allows for better targeted and earlier treatment than otherwise possible.

With this method it may be possible to screen and follow the migration behavior of tumor cells while elucidating the disturbance from cell-cell interactions. The method makes it possible to better analyze the interactions between the cells and the biomolecules, e.g., to analyze the impact the biomolecule provides to the cell migration. Furthermore, the method makes it possible to evaluate different biomolecules and evaluate pros and cons for different types of molecules in order to find out best possible alternative to predict metastasis potential of tumor cells.

The tumor cell may be applied within a migration corridor. The tumor cell may for example be applied in the beginning of the migration corridor, i.e., an end of the corridor. Alternatively, the cell may be applied just outside a migration corridor.

According to an example, the tumor cell is applied in an end of the migration corridor having the lowest nanoparticle concentration. Hereby, the cell is induced to migrate from an area with lower concentration of biomolecules to an area of higher concentration of biomolecules.

Alternatively, the cell may be applied in an area of intermediate nanoparticle concentration, i.e., an area located in between the highest concentration of nanoparticles and the lowest concentration of nanoparticles. By applying the cell applied in an area of intermediate nanoparticle concentration, both an attractive as well as a repellant effect of chemotactic biomolecules may be studied.

According to at least one example, the measuring and recording is performed by imaging and/or isotope analysis.

According to at least one example embodiment, the tumor cell is a breast cancer cell, a melanoma cancer cell, a prostate cancer cell, a colorectal cancer cell, or a lung cancer cell.

According to a third aspect of the disclosure, the use of the gradient-on-a-chip device according to the first aspect for quantification of cell migration and metastatic potential of tumor cells is provided.

According to at least one example embodiment, the tumor cell is a breast cancer cell, a melanoma cancer cell, a prostate cancer cell, a colorectal cancer cell, or a lung cancer cell. Non-limiting examples of features that may be measured and recorded are migration velocity, migration distance, migration motility, and migration direction.

Herein described is a gradient with multiple biomolecules and features that mimic conditions in vivo. This unlocks the ability to study tumor cells in real-time, with tumor microenvironment features and use this information to determine the metastatic potential of cell populations.

The device and the method can be used in a laboratory as a part of a diagnostic product on live patient tumor cells derived from a biopsy test at multiple points during a patient's cancer diagnostic, treatment, and monitoring.

### Definitions

Gradient is in this context meant concentration or density gradient, defined as the difference in the concentration or density of a substance between two areas.

A nano gradient layer is a surface covered with nanoparticles of which the distance between adjacent nanoparticles increase, or decrease, in a given direction from a given reference point.

The term nanoparticle is in this context meant particles of size between 1 and 100 nanometers (nm) in diameter.

The term nano gradient layer is surface covered with nanoparticles of which the distance between adjacent nanoparticles increase, or decrease, in a given direction from a given reference point.

The term continuous nano gradient layer is surface covered with nanoparticles of which the distance between adjacent nanoparticles increase, or decrease, continuously in a given direction from a given reference point.

The term gradient direction is in this context interpreted as the direction in which the distance between adjacent nanoparticles decrease or increase from a given reference point.

Generally, all terms used in the claims are to be interpreted according to their ordinary meaning in the technical field, unless explicitly defined otherwise herein. All references to "a/an/the [element, device, component, means, step, etc.]" are to be interpreted openly as referring to at least one instance of the element, device, component, means, step, etc., unless explicitly stated otherwise. The steps of any method disclosed herein do not have to be performed in the exact order disclosed, unless explicitly stated.

### BRIEF DESCRIPTION OF THE DRAWINGS

The disclosure will be described in more detail with reference to the appended schematic drawings, which show an example of a presently preferred embodiment of the disclosure.
**Fig. 1** illustrates a gradient-on-chip device according to at least one example embodiment of the inventive concept;
**Fig. 2** illustrates a gradient-on-chip device according to at least one example embodiment of the inventive concept;
**Fig. 3** illustrates in perspective view the gradient-on-ship device in Fig. 2;
**Fig. 4** illustrates different cell movement parameters (motility, migration and directional migration) on a xy-plane at different timepoints t₀ to tₙ of a cell;
**Fig. 5** illustrates cells migration directness with and without guiding structure;
**Fig. 6** illustrates cells migration directness with and without guiding structure.

### DETAILED DESCRIPTION

The present disclosure will now be described more fully hereinafter with reference to the accompanying drawings, in which currently preferred embodiments of the disclosure are shown. The present disclosure may, however, be embodied in many different forms and should not be construed as limited to the embodiments set forth herein; rather, these embodiments are provided for thoroughness and completeness, and to fully convey the scope of the disclosure to the skilled addressee. Like reference characters refer to like elements throughout.

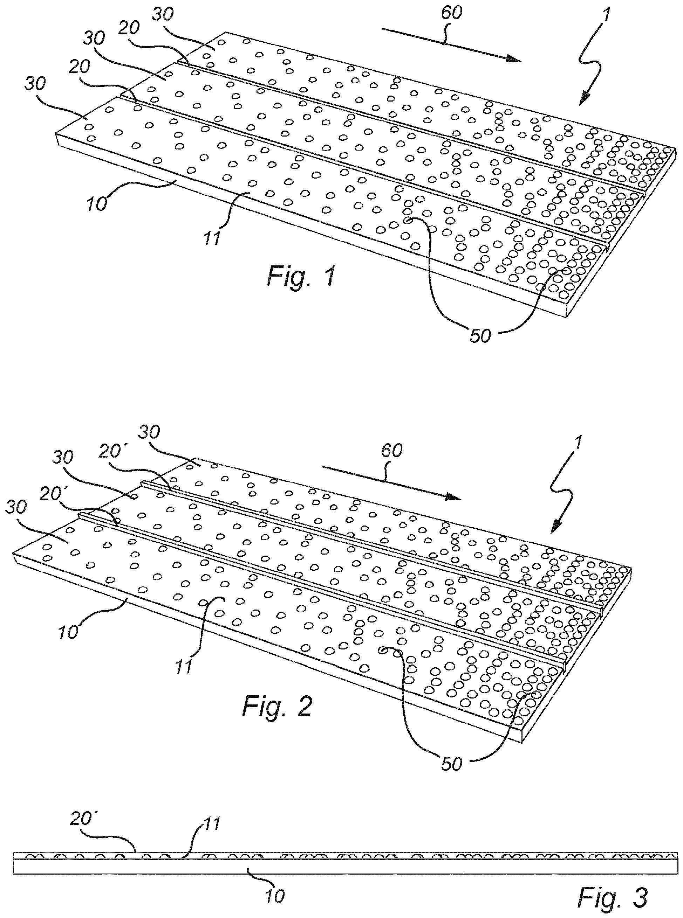

**Figs. 1** to **3** illustrate a gradient-on-chip device 1 according to at least one example embodiment of the inventive concept. The device 1 comprises a chip 10 of which upper part constitutes a chip surface 11. The function of the chip 10 is to support a nano gradient layer applied thereto. The chip 10 may consist of metal, polymer, or ceramics, such as glass. The chip 10 may for example, be a slide of glass or a slide of silicon.

The nano gradient layer extends over the entire chip surface 11 and comprises nanoparticles 50 provided in a gradient pattern. The nanoparticles 50 are for example, gold nanoparticles 50. An advantage with gold particles is that gold is inert and easy to link biomolecules to.

The nanoparticles 50 have a biomolecule applied to them. The biomolecule is attached to the nanoparticles 50 via a linker conjugated to the nanoparticles 50. The function of the linker is to enhance the adherence of the biomolecule to the nanoparticles 50, and is preferably located such that the cells easily can attract to them, such that the cells are affected by the linker. The linker is for example biotin.

The nano gradient layer has a gradient direction in an axis of the X-Y plane of the chip surface 11, for example in the X-axis. The chip surface 11 which the nano gradient is provided on, comprises at least one guiding structure 20 arranged in the gradient direction. The device 1 in Fig. 1 and Fig. 2, respectively, has two guiding structures 20, 20'. The guiding structures 20 in Fig. 1 are recessions pointing into the chip 10 whereas the guiding structures 20' in Fig. 2 are ridges extending out of the chip surface 11. These guiding structures 20, 20' divide the chip surface 11 and the nano gradient layer in three separate migration corridors 30. When the tumor cells are applied in a migration corridor 30 it is, thanks to the guiding structure 20, 20', prevented from reaching a tumor cell applied in a different migration corridor 30. This guiding structure 20, 20' thus prevents cell-cell interactions.

In order to measure cell migration, metastatic cells are provided within a migration corridor 30 of the device 1. The device 1 in Fig. 2 and Fig. 3 allows for three different cells to individually migrate in the gradient direction without disturbing each other if each one of them are placed in a separate migration corridor 30. It should be noted that the device 1 allows for measurement of more than one cell per migration corridor 30. In an alternative embodiment, the device 1 may comprise more than 100 migration corridors 30.

The cells are preferably repeatedly measured and recorded mapped in order to quantify migration, e.g., rate, distance, direction and/or motility. Mapping may be by, e.g., imaging and/or by isotope analysis.

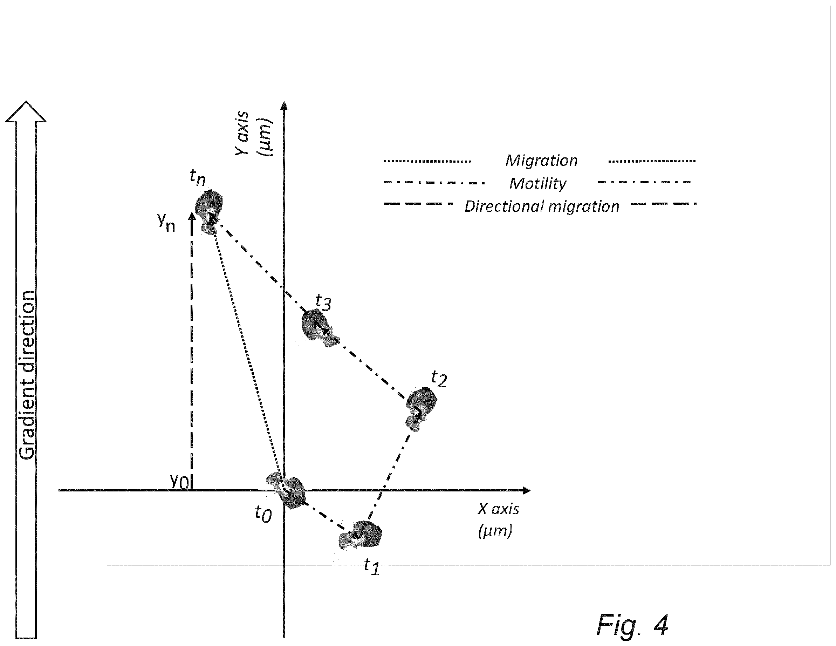

**Fig. 4** illustrates different cell movement parameters (motility, migration and directional migration) that can be measured on a xy-plane of gradient-on-a-chip device at different timepoints t₀ - tₙ. Motility is regarded as the total distance a cell travels over a period of time, e.g., t₀ to tₙ. Migration is measured as the direct distance between two coordinates after a certain time, e.g., t₀ and tₙ. Thus, motility ≥ migration. Migration directness is the ratio of migration and motility, indicating how direct a cell moves. Directional migration is defined as the distance a cell travels in a direction parallel to the orientation of the gradient after a certain time (tₙ), e.g., Y-Distance = yₙ-y₀, and represents the cell response to the gradient stimuli.

### Example 1

In the following example, cell migration was measured and compared on a gradient-on-chip device provided with migration corridors to the results obtained from a gradient-on-chip device without migration corridors. Using a gradient layer of nanoparticles (10 nm in diameter) (Cline NanoGradient, Cline Scientific, Sweden), conjugation of the biomolecules EGF to the nanoparticles and fibronectin in between the nanoparticles was conducted, scores of 1 to 2 µm depth and 1 to 5 µm width were generated on the left half of the nano-gradient surfaces in the same orientation as the biomolecule gradient direction using a diamond cuter under clean and humid condition to provide migration corridors. After the generation of the corridors, the nano-gradient surfaces were mounted in a Sarstedt 6 well plate, held in place using special clamps, and kept in PBS buffer solution supplemented with penicillin/streptomycin (1%, HyClone) until use. BT-549 (triple-negative breast cancer cells, ATCC) were cultured to 60 % confluency in culture medium (RPMI medium supplemented with insulin (1 µg/mL, GIBCO^{™}), Sodium-pyruvate (1 mM), non-essential amino acids (1%, HyClone), L-glutamine (2 mM, HyClone) and 10% fetal bovine serum (HyClone) until 24 hours prior to the experiment start point where the culture medium was replaced with the starvation medium (RPMI medium supplemented with Sodium-pyruvate, non-essential amino acids, L-glutamine and 1% fetal bovine serum). On the experiment day, cells were detached from the culture flask using TrypLE Express (GIBCO^{™}) according to the manufacturer's protocol and seeded on the nano-gradient surfaces at a concentration of 4000 cells/cm² in the starvation medium. The 6 well plates containing the nano-gradient surfaces and cells were placed in an incubator (37 C, 90% Rh, 5% CO₂) for 4 hours to allow cells to attach to the nano-gradient surfaces prior to real-time holographic imaging using a HoloMonitor^{®} M4 (phi, Sweden) placed in the incubator (37 C, 90% Rh, 5% CO₂). The segmentation and tracking analysis and data extraction were performed using the Hstudio^{™} software (PHI AB, Sweden).

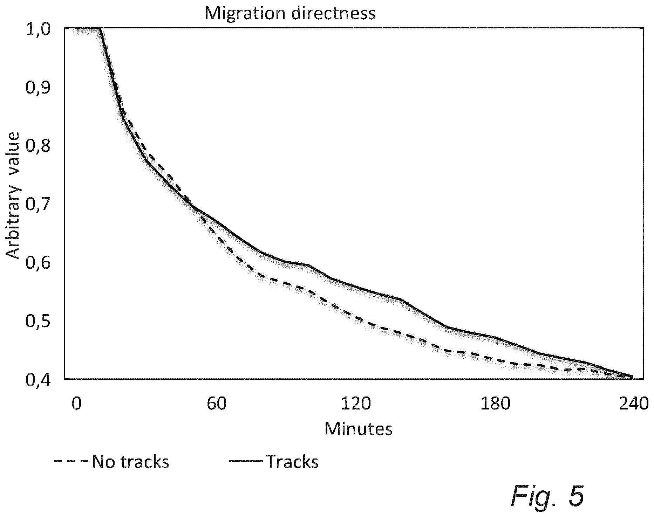

**Fig. 5** illustrates the calculated migration directness for the tracked cells over 4 hours. Cells (*n* = 204 tracked cells at t₀) initially show a higher migration directness on the gradient area with migration corridors during the first 3 hours compared to the cells (*n* = 128 tracked cells at t₀) on the gradient area with no migration corridors. It was noted that cells being present in the same area caused cells to interact with each other and not migrate in accordance with the gradient direction. The migration corridors limited the amount of cell-cell interaction. This resulted in tracked cells exposed to the area with scores to have a higher migration directness, that is, a more direct movement path.

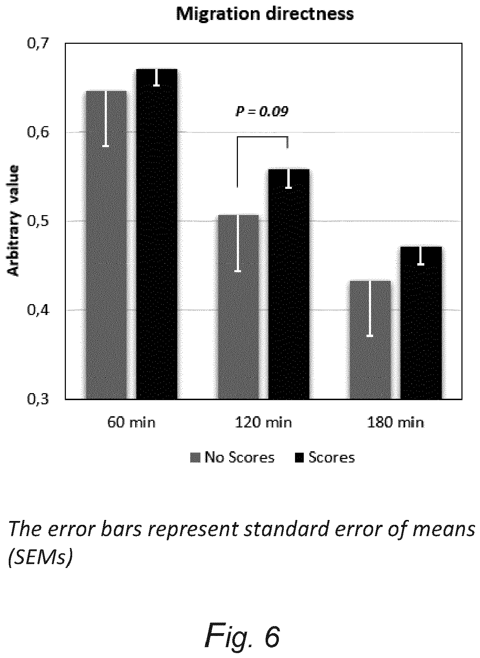

**Fig. 6** shows a comparison of the migration directness 1, 2, and 3 hours after the introduction of the cells to the nano gradient layer. The difference in migration directness between the two groups reaches its maximum after 2 hours (paired T-test, p = 0.09). These results suggest that the migration corridors minimize the cell-cell interactions, minimizing the impact of such events on cell migration and therefore allowing for better study of the cellular response to the gradient.

However, it was observed at around 4 hours that this effect on migration directness diminishes. Given the scores also limit the available space for cells on the nano-surface, over time, this resulted in increased cell-cell or cell-edge interactions when more than one cell is present. In addition, longer-term study of the cells introduces additional confounding factors such as cell division and change in motility speed which increases the probability of cell-cell interactions over time. Therefore, it is essential to design and induce scores (guiding structures) of proper dimensions to minimize cell-cell interaction over time.

## Claims

1. A gradient-on-a-chip device (1) for quantification of cell migration and metastatic potential of tumor cells, said device (1) comprising:
- a chip (10) having a chip surface (11);
- a nano gradient layer of nanoparticles (50) provided on said chip surface (11), said nano gradient layer having a gradient direction (60) along an axis of an X-Y plane of said chip surface (11); and
- biomolecules conjugated to said nanoparticles (50) by means of a linker linking together said biomolecule to said nanoparticles (50);
wherein
said chip surface (11) having at least one guiding structure (20, 20') arranged to guide said tumor cells in said gradient direction (60), said guiding structure (20, 20') extending in said gradient direction (60) and delineating a migration corridor (30) comprising said nano gradient layer, wherein said migration corridor (30) is 1 to 20 mm long and has a width in the range 20 to 500 µm, said migration corridor (30) having a length:width ratio of 200:1 to 20:1.

2. The device (1) according to claim 1, wherein said guiding structure (20, 20') is a ridge extending out of said chip surface (11), or wherein said guiding structure (20, 20') is a recession pointing into said chip surface (11).

3. The device (1) according to any one of claims 1 or 2, wherein said device (1) further comprises a chip surface (11) area void of nano gradient layer, and wherein said guiding structure (20, 20') is the boundary line between said migration corridor (30) and said chip surface (11) area void of nano gradient layer.

4. The device (1) according to any one of claims 1 to 3, wherein said guiding structure (20, 20') extends continuously along said chip surface (11); and/or wherein said migration corridor (30) has a substantially constant width along its extension direction.

5. The device (1) according to any one of claims 1 to 4, wherein said migration corridor (30) has a width in the range 50 to 200 µm.

6. The device (1) according to any one of claims 1 to 5, wherein said migration corridor (30) is 1 to 10 mm long.

7. The device (1) according to any one of claims 1 to 6, wherein said device (1) further comprises two or more migration corridors (30); preferably said two or more migration corridors (30) being of equal width; and/or wherein said device (1) further comprises two or more migration corridors (30) provided on top of each other.

8. The device (1) according to any one of claims 1 to 7, wherein said linker comprises the linker complex biotin/streptavidin.

9. The device (1) according to any one of claims 1 to 8, wherein the chip surface (11) between the nanoparticles (50) at least partly is coated by a coating agent; preferably the coating agent being ECM fibers, such as fibronectin, collagen, elastin, or laminin.

10. The device (1) according to any one of claims 1 to 9, wherein said nanoparticles (50) have a diameter in the range 1 to 100 nanometers (nm), such as in the range 3 to 30 nanometers (nm); and/or wherein said nanoparticles (50) are gold particles; preferably the gold particles having a diameter of 3 to 25 nm.

11. A method for quantification of cell migration and metastatic potential of tumor cells, said method comprising the steps of:
- applying at least one tumor cell to a gradient-on-a-chip device (1) according to any one of claims 1 to 10;
- repeatedly measuring and recording the cell migration of said tumor cell for a time period of 2 to 48 hours, preferably 3 to 24 hours, and most preferably 3 to 12 hours.

12. The method according to claim 11, wherein said measuring and recording is performed by imaging and/or isotope analysis.

13. The method according to any one of claims 11 to 12, wherein said tumor cell is a breast cancer cell, a melanoma cancer cell, a prostate cancer cell, a colorectal cancer cell, or a lung cancer cell.

14. The method according to any one of claims 11 to 13, wherein said biomolecule is Semaphorin-3E (Sema3E), Semaphorin-4D (Sema4D), Semaphorin-5a (Sema5a), C-C motif chemokine 27 (CCL27), C-C motif chemokine 38 (CCL38), C-C motif chemokine 48 (CCL48), C-C motif chemokine 58 (CCL58), C-C motif chemokine 12 (CCL12), C-C motif chemokine 199 (CCL199), C-C motif chemokine 21 (CCL21), C-C motif chemokine 22 (CCL22), C-C motif chemokine 25 (CCL25), C-X-C motif chemokine 5 (CXCL5), C-X-C motif chemokine 8 (CXCL8) (IL-8), C-X-C motif chemokine 9 (CXCL9), C-X-C motif chemokine 10 (CXCL10), C-X-C motif chemokine 12 (CXCL12), C-X-C motif chemokine 13 (CXCL13), C-X-C motif chemokine 14 (CXCL14), Interleukin-11 (IL-11), Fibroblast growth factor (FGF), Platelet-derived growth factor (PDGF), Placenta growth factor (PIGF), Hepatocyte growth factor (HGF), HB-EGF (Heparin-binding, EGF-like), Slit homolog 2 protein (Slit2), Vascular endothelial growth factor a (Vegf-a), Vascular endothelial growth factor b (Vegf-b), Vascular endothelial growth factor c (Vegf-c), Ephrin type-A receptor 2 (EphA2) (Eph - receptor), or Ephrin type-B receptor 4 (EphB4); preferably said biomolecule being epidermal growth factor (EGF)

15. Use of the gradient-on-a-chip device (1) according to any one of claims 1 to 10 for quantification of cell migration and metastatic potential of tumor cells.

## Patentansprüche

1. Gradient-on-a-Chip-Vorrichtung (1) zur Quantifizierung der Zellmigration und des metastatischen Potenzials von Tumorzellen, wobei die Vorrichtung (1) umfasst:
- einen Chip (10) mit einer Chipoberfläche (11);
- eine Nanogradientenschicht aus Nanopartikeln (50), die auf der Chipoberfläche (11) vorgesehen ist, wobei die Nanogradientenschicht eine Gradientenrichtung (60) entlang einer Achse einer X-Y-Ebene der Chipoberfläche (11) aufweist; und
- Biomoleküle, die mit den Nanopartikeln (50) mittels eines Linkers konjugiert sind, der das Biomolekül mit den Nanopartikeln (50) verbindet;
wobei
die Chipoberfläche (11) mindestens eine Führungsstruktur (20, 20') aufweist, die so angeordnet ist, dass sie die Tumorzellen in der Gradientenrichtung (60) führt, wobei sich die Führungsstruktur (20, 20') in der Gradientenrichtung (60) erstreckt und einen Migrationskorridor (30) abgrenzt, der die Nano-Gradientenschicht umfasst, wobei der Migrationskorridor (30) 1 bis 20 mm lang ist und eine Breite im Bereich von 20 bis 500 µm aufweist, wobei der Migrationskorridor (30) ein Verhältnis von Länge zu Breite von 200:1 bis 20:1 aufweist.

2. Vorrichtung (1) nach Anspruch 1, wobei die Führungsstruktur (20, 20') eine aus der Chipoberfläche (11) herausragende Rippe ist, oder wobei die Führungsstruktur (20, 20') eine in die Chipoberfläche (11) weisende Vertiefung ist.

3. Vorrichtung (1) nach einem der Ansprüche 1 oder 2, wobei die Vorrichtung (1) ferner einen Bereich der Chipoberfläche (11) ohne Nano-Gradientenschicht aufweist, und wobei die Führungsstruktur (20, 20') die Grenzlinie zwischen dem Migrationskorridor (30) und dem Bereich der Chipoberfläche (11) ohne Nano-Gradientenschicht ist.

4. Vorrichtung (1) nach einem der Ansprüche 1 bis 3, wobei sich die Führungsstruktur (20, 20') kontinuierlich entlang der Chipoberfläche (11) erstreckt; und/oder wobei der Migrationskorridor (30) eine im Wesentlichen konstante Breite entlang seiner Erstreckungsrichtung aufweist.

5. Vorrichtung (1) nach einem der Ansprüche 1 bis 4, wobei der Migrationskorridor (30) eine Breite im Bereich von 50 bis 200 µm aufweist.

6. Vorrichtung (1) nach einem der Ansprüche 1 bis 5, wobei der Migrationskorridor (30) 1 bis 10 mm lang ist.

7. Vorrichtung (1) nach einem der Ansprüche 1 bis 6, wobei die Vorrichtung (1) ferner zwei oder mehr Migrationskorridore (30) umfasst; vorzugsweise sind die zwei oder mehr Migrationskorridore (30) gleich breit; und/oder wobei die Vorrichtung (1) ferner zwei oder mehr übereinander angeordnete Migrationskorridore (30) umfasst.

8. Vorrichtung (1) nach einem der Ansprüche 1 bis 7, wobei der Linker den Linker-Komplex Biotin/Streptavidin umfasst.

9. Vorrichtung (1) nach einem der Ansprüche 1 bis 8, wobei die Chipoberfläche (11) zwischen den Nanopartikeln (50) zumindest teilweise mit einem Beschichtungsmittel beschichtet ist; wobei das Beschichtungsmittel vorzugsweise ECM-Fasern, wie Fibronectin, Kollagen, Elastin oder Laminin, ist.

10. Vorrichtung (1) nach einem der Ansprüche 1 bis 9, wobei die Nanopartikel (50) einen Durchmesser im Bereich von 1 bis 100 Nanometern (nm), wie beispielsweise im Bereich von 3 bis 30 Nanometern (nm), aufweisen; und/oder wobei die Nanopartikel (50) Goldpartikel sind; vorzugsweise weisen die Goldpartikel einen Durchmesser von 3 bis 25 nm auf.

11. Verfahren zur Quantifizierung der Zellmigration und des metastatischen Potenzials von Tumorzellen, wobei das Verfahren die folgenden Schritte umfasst:
- Aufbringen mindestens einer Tumorzelle auf eine Gradient-on-a-Chip-Vorrichtung (1) nach einem der Ansprüche 1 bis 10;
- wiederholtes Messen und Aufzeichnen der Zellmigration der Tumorzelle über einen Zeitraum von 2 bis 48 Stunden, vorzugsweise 3 bis 24 Stunden und am meisten bevorzugt 3 bis 12 Stunden.

12. Verfahren nach Anspruch 11, wobei das Messen und Aufzeichnen durch Bildgebung und/oder Isotopenanalyse erfolgt.

13. Verfahren nach einem der Ansprüche 11 bis 12, wobei die Tumorzelle eine Brustkrebszelle, eine Melanomkrebszelle, eine Prostatakrebszelle, eine Dickdarmkrebszelle oder eine Lungenkrebszelle ist.

14. Verfahren nach einem der Ansprüche 11 bis 13, wobei das Biomolekül Semaphorin-3E (Sema3E), Semaphorin-4D (Sema4D), Semaphorin-5a (Sema5a), C-C-Motiv Chemokin 27 (CCL27), C-C-Motiv Chemokin 38 (CCL38), C-C-Motiv Chemokin 48 (CCL48), C-C-Motiv Chemokin 58 (CCL58), C-C-Motiv-Chemokin 12 (CCL12), C-C-Motiv-Chemokin 199 (CCL199), C-C-Motiv-Chemokin 21 (CCL21), C-C-Motiv-Chemokin 22 (CCL22), C-C-Motiv-Chemokin 25 (CCL25), C-X-C-Motiv-Chemokin 5 (CXCL5), C-X-C-Motiv-Chemokin 8 (CXCL8) (IL-8), C-X-C-Motiv-Chemokin 9 (CXCL9), C-X-C-Motiv-Chemokin 10 (CXCL10), C-X-C-Motiv-Chemokin 12 (CXCL12), C-X-C-Motiv-Chemokin 13 (CXCL13), C-X-C-Motiv-Chemokin 14 (CXCL14), Interleukin-11 (IL-11), Fibroblasten-Wachstumsfaktor (FGF), Plättchen-Wachstumsfaktor (PDGF), Plazenta-Wachstumsfaktor (PlGF), Hepatozyten-Wachstumsfaktor (HGF), HB-EGF (Heparin-bindend, EGF-ähnlich), Slit-Homolog-2-Protein (Slit2), vaskulärer endothelialer Wachstumsfaktor a (Vegf-a), vaskulärer endothelialer Wachstumsfaktor b (Vegf-b), vaskulärer endothelialer Wachstumsfaktor c (Vegf-c), Ephrin-Typ-A-Rezeptor 2 (EphA2) (Eph - Rezeptor) oder Ephrin-Typ-B-Rezeptor 4 (EphB4) ist; vorzugsweise ist das Biomolekül epidermaler Wachstumsfaktor (EGF).

15. Verwendung der Gradient-on-a-Chip-Vorrichtung (1) nach einem der Ansprüche 1 bis 10 zur Quantifizierung der Zellmigration und des metastatischen Potenzials von Tumorzellen.

## Revendications

1. Dispositif de gradient sur puce (1) pour la quantification de la migration cellulaire et du potentiel métastatique des cellules tumorales, ledit dispositif (1) comprenant :
- une puce (10) ayant une surface de puce (11) ;
- une couche de nanoparticules en gradient (50) disposée sur ladite surface de puce (11), ladite couche en gradient ayant une direction de gradient (60) le long d'un axe d'un plan X-Y de ladite surface de puce (11) ; et
- des biomolécules conjuguées auxdites nanoparticules (50) au moyen d'un linker reliant ladite biomolécule auxdites nanoparticules (50) ;
dans lequel
ladite surface de puce (11) comporte au moins une structure de guidage (20, 20') conçue pour guider lesdites cellules tumorales dans ladite direction de gradient (60), ladite structure de guidage (20, 20') s'étendant dans ladite direction de gradient (60) et délimitant un couloir de migration (30) comprenant ladite couche en gradient, dans lequel ledit couloir de migration (30) a une longueur de 1 à 20 mm et une largeur de 20 à 500 µm, ledit couloir de migration (30) ayant un rapport longueur:largeur de 200:1 à 20:1.

2. Le dispositif (1) selon la revendication 1, dans lequel ladite structure de guidage (20, 20') est une arête s'étendant hors de ladite surface de puce (11), ou dans lequel ladite structure de guidage (20, 20') est un renfoncement pointant dans ladite surface de la puce (11).

3. Le dispositif (1) selon l'une quelconque des revendications 1 ou 2, dans lequel ledit dispositif (1) comprend en outre une zone de surface de puce (11) dépourvue de couche en gradient, et dans lequel ladite structure de guidage (20, 20') est la ligne de démarcation entre ledit couloir de migration (30) et ladite zone de surface de puce (11) dépourvue de couche en gradient.

4. Le dispositif (1) selon l'une quelconque des revendications 1 à 3, dans lequel ladite structure de guidage (20, 20') s'étend de manière continue le long de ladite surface de puce (11) ; et/ou dans lequel ledit couloir de migration (30) a une largeur sensiblement constante le long de sa direction d'extension.

5. Le dispositif (1) selon l'une quelconque des revendications 1 à 4, dans lequel ledit couloir de migration (30) a une largeur comprise entre 50 et 200 µm.

6. Le dispositif (1) selon l'une quelconque des revendications 1 à 5, dans lequel ledit couloir de migration (30) a une longueur de 1 à 10 mm.

7. Le dispositif (1) selon l'une quelconque des revendications 1 à 6, dans lequel ledit dispositif (1) comprend en outre deux ou plusieurs couloirs de migration (30) ; de préférence, dans lequel lesdits deux ou plusieurs couloirs de migration (30) sont de largeur égale ; et/ou dans lequel ledit dispositif (1) comprend en outre deux ou plusieurs couloirs de migration (30) superposés.

8. Le dispositif (1) selon l'une quelconque des revendications 1 à 7, dans lequel ledit linker comprend le complexe de linker biotine/streptavidine.

9. Le dispositif (1) selon l'une quelconque des revendications 1 à 8, dans lequel la surface de puce (11) entre les nanoparticules (50) est au moins partiellement recouverte d'un agent de revêtement ; de préférence, dans lequel l'agent de revêtement est constitué de fibres MEC, telles que la fibronectine, le collagène, l'élastine ou la laminine.

10. Le dispositif (1) selon l'une quelconque des revendications 1 à 9, dans lequel lesdites nanoparticules (50) ont un diamètre compris entre 1 et 100 nanomètres (nm), tels que entre 3 et 30 nanomètres (nm) ; et/ou dans lequel lesdites nanoparticules (50) sont des particules d'or ; de préférence, dans lequel les particules d'or ont un diamètre compris entre 3 et 25 nm.

11. Méthode de quantification de la migration cellulaire et du potentiel métastatique des cellules tumorales, comprenant les étapes suivantes :
- appliquer au moins une cellule tumorale à un dispositif de gradient sur puce (1) selon l'une quelconque des revendications 1 à 10 ;
- mesurer et enregistrer de manière répétée la migration cellulaire de ladite cellule tumorale pendant une période de 2 à 48 heures, de préférence de 3 à 24 heures, et de préférence encore de 3 à 12 heures.

12. Méthode selon la revendication 11, dans laquelle la mesure et l'enregistrement sont effectués par imagerie et/ou analyse isotopique.

13. Méthode selon l'une quelconque des revendications 11 à 12, dans laquelle la cellule tumorale est une cellule cancéreuse du sein, une cellule cancéreuse du mélanome, une cellule cancéreuse de la prostate, une cellule cancéreuse colorectale ou une cellule cancéreuse du poumon.

14. Méthode selon l'une quelconque des revendications 11 à 13, dans laquelle ladite biomolécule est la sémaphorine-3E (Sema3E), la sémaphorine-4D (Sema4D), la sémaphorine-5a (Sema5a), la chimiokine 27 à motif C-C (CCL27), la chimiokine 38 à motif C-C (CCL38), la chimiokine 48 à motif C-C (CCL48), la chimiokine 58 à motif C-C (CCL58), la chimiokine 12 à motif C-C (CCL12), la chimiokine 199 à motif C-C (CCL199), la chimiokine 21 à motif C-C (CCL21), la chimiokine 22 à motif C-C (CCL22), la chimiokine 25 à motif C-C (CCL25), la chimiokine 5 à motif C-X-C (CXCL5), la chimiokine 8 à motif C-X-C (CXCL8) (IL-8), la chimiokine 9 à motif C-X-C (CXCL9), la chimiokine 10 à motif C-X-C (CXCL10), la chimiokine 12 à motif C-X-C (CXCL12), la chimiokine 13 à motif C-X-C (CXCL13), la chimiokine 14 à motif C-X-C (CXCL14), l'interleukine-11 (IL-11), un facteur de croissance des fibroblastes (FGF), un facteur de croissance dérivé des plaquettes (PDGF), un facteur de croissance placentaire (PlGF), un facteur de croissance des hépatocytes (HGF), HB-EGF (liaison à l'héparine, de type EGF), une protéine Slit homologue 2 (Slit2), un facteur de croissance de l'endothélium vasculaire a (Vegf-a), un facteur de croissance de l'endothélium vasculaire b (Vegf-b), un facteur de croissance de l'endothélium vasculaire c (Vegf-c), un récepteur 2 de l'éphrine de type A (EphA2) (-récepteur Eph), ou un récepteur 4 de l'éphrine de type B (EphB4) ; de préférence, dans laquelle ladite biomolécule est le facteur de croissance épidermique (EGF).

15. Utilisation du dispositif de gradient sur puce (1) selon l'une quelconque des revendications 1 à 10 pour quantifier la migration cellulaire et le potentiel métastatique des cellules tumorales.
